# EUROPEAN PATENT APPLICATION

(11) **EP 1 482 053 A2**
(43) Date of publication of application: **01.12.2004**
(21) Application number: 04075824.5
(22) Date of filing: 21.12.1990
(51) Int. Cl.: C12N 15/90, C12N 5/10, C12N 15/85, C12N 15/67

(54) **Eukaryotic host cell line in which an endogenous gene has been activated, and uses thereof**

(30) Priority: 22.12.1989 US 454783
(62) Divisional of application: 03102675.0
(71) Applicant: Applied Research Systems ARS Holding N.V., Curacao (AN)
(72) Inventor: Chappel, Scott C., Milton, Massachussetts (US)
(74) Representative: Winckels, Johannes Hubertus F.

(57) **Abstract**

The invention relates to the a eukaryotic host cell line, obtained by the following process:
- inserting by homologous recombination a DNA construct comprising at least a DNA regulatory segment F which activates or modifies expression of a preselected gene in a predetermined eukaryotic host cell line when operatively linked thereto, a DNA targeting segment homologous to a region of the genome within or proximal to the preselected gene within the host cell line, and a selectable marker;
- chemical selection, and
- monitoring for production of the specific gene product of the newly activated gene in the eukaryotic host cell line.

The cell line is used for production of a specific gene product.

SEQUENCE LISTING

<110>  Applied Research Systems ARS Holding N.V.

<120>  Eukaryotic host cell line in which an endogenous gene has been activated,
and uses thereof

<130>  EP 230 DIV2

<140>  EP03102675.0

<141>  2003-08-29

<150>  EP96203412.0

<151>  1990-12-21

<160>  3

<170>  PatentIn version 3.1

<210>  1

<211>  999

<212>  DNA

<213>  Rattus norvegicus

<400>  1
ggcacgcctc tgaatgtgga aaggacactt atgagctctg tggtctttcc ctctgattta      60

gcatgaatgc tgtcgttctc ttttccgtgc ttttcgctct tgcttgtggg caagtgtcat     120

cgttttgtat tcccactgag tatatgatgt acgtggacag gagagagtgt gcctactgcc     180

tgaccatcaa caccaccatc tgcgctgggt attgtatgac acgggtatgt tggtcactgc     240

gtttctttta gctgtaaatt gtacaggtct aaagttgtct gttaatattt tagaaaggaa     300

gtgggataaa tcatagtctc ctctttggga agccaagcac actgctttca gaattataat     360

tatgtcattc tacacagaaa aagtacagat acattgtaac agtttaccct aaagtgtttg     420

ttctgctcaa tggtagatga gaagaaagtg tccttttttg tctctgaggg gttaagtgta     480

gatgtgtggg taacagagct caggagtcct ttaagatcat caggaaacaa agggatatta     540

gtcattctat tacactaagt tgcatgcagt ttatcatgtt aagatctctt ttcttccaca     600

ggatatcaat ggcaaactgt ttcttcccaa gtacgcactc tctcaggatg tctgtacata 660

cagagacttc acctacagaa cggtggaaat accgggatgc ccacaccatg ttgctcctta 720

tttctcctac cccgttgccc tgagctgcaa gtgtggcaag tgtaacactg actacagcga 780

ctgtacacac gaggctgtca aaaccaacta ctgcaccaag ccacagacat tctatctggg 840

gggattttct ggttaactgt aatggcaatg caatctggtt aaatgtgttt acctggaata 900

gaactaataa aatatcattg atatgtcttg cctgccattt aatccatagg cacatccaca 960

aggcattaga gagcttacac aactttagaa gcagaggcg 999

<210> 2

<211> 23

<212> DNA

<213> Rattus norvegicus

<400> 2
agtatatgat gtacgtggac agg 23

<210> 3

<211> 24

<212> DNA

<213> Rattus norvegicus

<400> 3
cacttgccac acttgcagct cagg 24

## Description

### FIELD OF INVENTION

The present invention relates to a process for the modification of the expression characteristics of a gene which is naturally present within the genome of a stable cell line or cloned microorganism. In the preferred embodiment, the present invention relates to a process for the activation and expression of a gene that is present within a stable cell line and normally transcriptionally silent or inert. As a result, the protein product of that gene is expressed. This phenomenon occurs without transfecting the cell with the DNA that encodes the product. Rather, the resident gene coding for the desired product is identified within a cell and activated by inserting an appropriate regulatory segment through a technique called homologous recombination. Positive and/or negative selectable markers can also be inserted to aid in selection of the cells in which proper homologous recombination events have occurred. As an additional embodiment, a specified gene can be amplified for enhanced expression rates, whether that gene is normally transcriptionally silent and has been activated by means of the present invention, or endogenously expresses product.

### BACKGROUND OF THE INVENTION

It is well known that each cell within an organism contains the genetic information that encodes all of the proteins found within that organism. However, only a very small percentage of the genes present within a given cell type is actually transcribed. The intracellular mechanisms that regulate the array of genes to be transcribed are now understood. Cell specific proteins present within the nucleus interact with DNA regulatory segments that are linked with particular genes. This interaction of nuclear proteins with DNA regulatory sequences is required for gene transcription. This results in mRNA biosynthesis and ultimate expression of the encoded protein (Mitchell and Tjian, Science, 245:371,1989).

These DNA regulatory segments or elements for each gene lie upstream from and, in some cases, within or even downstream of the coding regions. Through an interaction with cell specific nuclear proteins, DNA regulatory segments affect the ability of RNA polymerase, the rate limiting enzyme in protein expression, to gain access to the body of the gene and synthesize a mRNA transcript. Thus, these DNA segments and the resident nuclear proteins play a critical role in the regulation of expression of specific genes (Johnson and McKnight, Ann. Rev. Biochem., 58:799, 1989).

The DNA regulatory segments are binding sites for the nuclear proteins. These nuclear proteins attach to the DNA helix and apparently alter its structure to make the desired gene available for RNA polymerase recognition, which facilitates gene transcription. The expression of these cell specific regulatory proteins determines which genes will be transcribed within a cell and the rate at which this expression will occur. As an example of the specificity of this system, pituitary cells but not liver cells express pituitary proteins, even though the genes for the pituitary proteins are present within all liver cells. Nuclei of the liver cells do not contain the specific DNA binding proteins which interact with the elements of pituitary gene resident within the liver cells.

Current Methods Employed to Express Proteins Using Recombinant DNA Technoloqy

With the knowledge that specific DNA regulatory sequences are required to activate gene transcription within a particular cell type, scientists have expressed foreign genes within a particular cell type through genetic engineering. In general, DNA regulatory segments that are recognized by the cell's nuclear proteins are placed upstream from the coding region of a foreign gene to be expressed. In this way, after insertion into the cell, foreign DNA may be expressed since the cell's nuclear regulatory proteins now recognize these DNA regulatory sequences. This technology has been employed to produce proteins that have been difficult to obtain or purify from natural sources by traditional purification strategies.

In addition to the recognizable DNA sequences and the gene of interest, a selectable marker is attached to the DNA construction. In this way, only the cells that have taken up the DNA survive following culture in a selectable medium. For example, the gene for neomycin resistance may be included in the expression vector. Following transfection, cells are cultured in G418, a neomycin antibiotic that is lethal to mammalian cells. If however, the cells have acquired the neomycin resistance gene, they will be able to withstand the toxic effects of the drug. In this way, only the cells that have taken up the transfected DNA are maintained in culture. It is understood that any selectable marker could be used as long as it provided for selection of cells that had taken up the transfected DNA. It is further understood that there is no criticality as to the specific location of the inserted genetic material within the cell. It is only important that it be taken up somewhere within the nucleus as both the regulatory segment and the foreign gene (as well as the selectable marker) are inserted together.

### Deficiencies in the Current Methods of Gene Expression

While the above techniques have been instrumental in exploiting the power of genetic engineering, they have not always been the most efficient methods to express genes. This is due to the fact that insertion of DNA into the nucleus of a cell line is usually accomplished through a technique known as transfection. DNA that has been engineered for expression in the cell line of interest is precipitated and the cell membrane is solubilized to allow entry of the DNA. As indicated above, the exact site into which the DNA incorporates into the genome is never predictable; indeed the DNA may remain episomal (not integrated into the genome). This results in the unpredictability of both the level of expression of the protein produced and the stability of the cell line.

A second shortcoming of this technique is the fact that the construction of the expression vector is extremely difficult when the gene of interest is relatively large (greater than 5-10 kilobases). Many of the proteins expressed by recombinant DNA technology have been encoded by cDNAs rather than much larger genomic clones. This is done to reduce the overall size of the insert. While the use of cDNAs makes genetic engineering more convenient, rates of gene transcription and protein production may suffer as a result. It has recently been shown that expression levels are sometimes greatly enhanced through the use of genomic rather than cDNA inserts (Brinster et al., Proc. Natl. Acad. Sci., 85:836-840, 1988, and Chung and Perry, Mol. Cell. Biol., 9:2075- 2082, 1989). Although the mechanisms responsible for this observation are not well understood, it is known that in certain situations enhancer elements present within introns can improve the transcriptional efficiency of the gene. There is also evidence that introns, or the splicing events which result from the presence of introns, may have an effect on the RNA processing events which follow the initiation of transcription (Buchman and Berg, Mol. Cell. Biol., 8:43954405, 1988). This may stabilize the transcript thereby improving the rate of mRNA accumulation. In the above-cited Brinster et al paper, it is also postulated that the position of the introns within the gene may be important for phasing of nucleosomes relative to the promoter. The influence of various regulatory elements on transcription of eukaryotic genes is discussed in Khoury et al, Cell, 33:313-14 (1983), Maniatis et al, Science, 236:1237-45 (1987) and Muller et al, Eur. J. Biochem., 176:485-95 (1988).

Thirdly, to gain entry into the nucleus, the transfected DNA, including the entire coding region of the foreign gene, must traverse the cytoplasm following entry through the permeabilized plasma membrane of the cell. During that time, the DNA may come in contact with lysosomal enzymes which may alter or completely destroy the integrity of the DNA. Thus, the coding region of the DNA may not be identical to that which was transfected.

The novel method of gene activation and/or expression modification that we describe below cannot result in the production of mutant forms of the desired protein, since the coding region of the desired gene is not subjected to enzymatic modifications.

In summary, a large amount of the DNA transfected into the cell using traditional techniques, and particularly the coding region thereof, will not be faithfully transcribed. It may be degraded prior to entry into the nucleus, enzymatically perturbed so that it will not encode the entire desired protein or it may not contain all of the necessary regulatory segments to allow for transcription. It may be inserted into a portion of the genome that prevents transcription. If the cDNA is transcribed, the protein of interest may not be produced efficiently due to the omission of introns which may contain enhancers or enable efficient mRNA processing. Finally, it may remain episomal, promote protein production but be unstable as the cell population grows through cell division.

It would be most desirable to develop a method of induct ion of gene expression that would produce a cell line that has incorporated the positive attributes of the existing methods but somehow circumvents the unattractive features. It would further be desirable to be able to express or modify endogenous expression of particular genes in the cell type of choice. It is further desired to be able to take advantage of the potential benefits that may be afforded by a complete genomic sequence which may include cryptic transcriptional enhancers that may reside within introns, by appropriate placement of introns for proper nucleosome phasing or by more efficient mRNA processing events. These advantages are ordinarily not enjoyed in recombinant DNA expression methods due to the size of the gene. If one were able to express a gene that is already resident in the genome, i.e., an endogenous gene, cell line stability and expression rates would become more consistent and predictable.

### SUMMARY OF THE INVENTION

Accordingly, it is an object of the present invention to eliminate the above-noted deficiencies in the prior art.

It is another object of the present invention to provide a method of regulation and/or amplification of gene expression that incorporates the positive attributes of recombinant gene technology but circumvents the unattractive features.

It is a further object of the present invention to provide a method for expressing specific genes present but normally transcriptionally silent in a cell line of choice.

It is yet a further object of the present invention to provide a method for expressing proteins which takes full advantage of complete genomic sequences that are responsible for mRNA accumulation and/or transcription.

It is still another object of the present invention to provide a method of modifying the expression characteristics of a gene of interest by inserting DNA regulatory segments and/or amplifying segments into the genome of a stable cell line or cloned microorganism upstream of, within, or otherwise proximal to the native gene of interest.

It is still a further object of the present invention to provide a method for modifying the expression characteristics of a gene which is naturally present within the genome of a stable cell line or cloned microorganism and at the same time insert characteristics which will aid in the selection of cells which have been properly modified.

It is yet another object of the present invention to provide a genome having therein, proximal to the coding region or exons of a gene of interest, a regulatory or amplifying segment which does not naturally appear thereat.

It is another object of the present invention to provide DNA constructs which can be used for accomplishing the homologous recombination methods of the present invention.

It is a further object of the present invention to provide cell lines and microorganisms which include the genomes in accordance with the present invention.

These and other objects of the present invention are accomplished by means of the technique of homologous recombination, by which one of ordinary skill in this art can cause the expression and, preferably, amplification of resident, albeit transcriptionally silent genes. By this technique, one can also modify the expression characteristics of a gene which is naturally present, but not necessarily silent or inert, within the genome of a stable cell line, such as, for example, to make the expression conditional, i.e., repressible or inducible, or to enhance the rate of expression.

The present invention provides a method of modifying the expression characteristics of a gene within the genome of a cell line or microorganism. A DNA construct is inserted into that genome by the technique of homologous recombination. The construct includes a DNA regulatory segment capable of modifying the expression characteristics of any gene to which it is operatively linked within the host cell line or microorganism, as well as a targeting segment homologous to a region of the genome at which it is desired for the DNA regulatory segment to be inserted. The construct and insertion technique is designed to cause the new DNA regulatory segment to be operatively linked to the gene of interest. Thus, without necessarily inserting any new coding exons, the expression characteristics of that gene are modified. In the preferred embodiment, the gene is one which is normally transcriptionally silent or inert within the host cell line or microorganism and, by means of the DNA regulatory region, which is targeted directly to the appropriate position with respect to that gene by means of homologous recombination, that gene is thereby activated for expression of its gene product.

The DNA construct preferably includes two targeting segments which, while separated from one another in the construct by those elements to be inserted into the genome, are preferably contiguous in the native gene. Preferably, in the DNA construct, at least the upstream DNA targeting segment is homologous to a non-coding region of the gene. Preferably, the downstream DNA targeting segment includes a portion of the preselected gene.

The construct further preferably includes at least one expressible selectable marker gene, such as the gene providing neomycin resistance. This marker gene, including a promoter therefor, is also disposed between the two targeting regions of the construct.

In another embodiment, the construct includes an expressible amplifiable gene in order to amplify expression of the gene of interest. This gene, including a promoter therefor, is also disposed between the two targeting regions of the construct. In some cases the selectable marker and the amplifiable marker may be the same.

In a further embodiment of the present invention, the DNA construct includes a negative selectable marker gene which is not expressed in cells in which the DNA construct is properly inserted. This negative selectable marker gene is disposed outside of the two targeting regions so as to be removed when the construct is properly combined into the gene by homologous recombination. An example of such a negative selectable marker gene is the Herpes Simplex Virus thymidine kinase gene.

In yet a further embodiment, it is possible to modify the expression characteristics of a specific gene which already expresses a product in the cell line or microorganism of interest. This can be accomplished by inserting by homologous recombination a DNA construct which includes (1) an expressible amplifiable gene which increases the copy number of the gene of interest when the cell line or microorganism is subjected to -amplification conditions and/or (2) a promoter/enhancer element (or other regulatory element) which modifies the expression of the gene of interest such as, for example, by increasing the rate of transcription, increasing translation efficiency, increasing mRNA accumulation, making the expression inducible, etc. The gene expression which is modified in this manner may be natural expression or expression which has been caused by previous genetic manipulation of the cell line or microorganism. The previous genetic manipulation may have been by conventional techniques or by means of homologous recombination in accordance with the present invention. In the latter case, the DNA insertion which results in the modification of expression characteristics may be accomplished as part of the same genetic manipulation which results in expression of the gene or may be performed as a subsequent step.

The present invention also includes the constructs prepared in accordance with the above discussion as well as the genomes which have been properly subjected to homologous recombination by means of such constructs and the cell lines and microorganisms including these genomes.

Moreover, a process for preparation of the desired product by culturing the transformed cells according to the present invention is also included.

The present invention also includes the use of a DNA construct of the invention in activating the expression of a preselected normally transcriptionally silent gene in a predetermined eukaryotic host cell line.

The present invention also includes a eukaryotic host cell line obtained by the following process:
- inserting by homologous recombination a DNA construct comprising at least a DNA regulatory segment F which activates or modifies expression of a preselected gene in a predetermined eukaryotic host cell line when operatively linked thereto, a DNA targeting segment homologous to a region of the genome within or proximal to the preselected gene within the host cell line, and a selectable marker;
- chemical selection, and
- monitoring for production of the specific gene product of the newly activated gene in the eukaryotic host cell line.

The use of such a eukaryotic host cell line is also within the present invention.

The invention further relates to the use of a DNA construct for activation of a gene in a eukaryotic cell line by homologous recombination and expression of the activated gene, wherein the gene is a normally transcriptionally silent gene, the DNA construct comprising
- a DNA regulatory segment F which activates expression of a preselected gene in a predetermined eukaryotic host cell line when operatively linked thereto; and
- at least a DNA targeting segment homologous to a region of the genome within or proximal to the preselected gene within the host cell line; and
- a selectable marker.

The invention further relates to a method for producing a gene product comprising the steps of:
- culturing a eukaryotic cell that has within its genome a normally transcriptionally silent endogenous gene of interest linked in an operative way to a heterologous regulatory segment that stimulates transcription of the gene of interest; and
- monitoring for production of the gene product.

### BRIEF DESCRIPTION OF THE DRAWINGS

Fig. 1 shows a general outline of a DNA construct in accordance with the present invention.
Fig. 2A shows the mode of integration of the DNA construct into the genome in the event of non-homologous or random recombination.
Fig. 2B shows the mode of integration of the DNA construct in the genome in the event of homologous recombination.
Fig. 3 shows the construction of a preferred homologous recombination vector in accordance with the present invention.
Fig. 4 shows the mode of integration of a circular piece of DNA by homologous recombination when only a single targeting piece of DNA is employed.

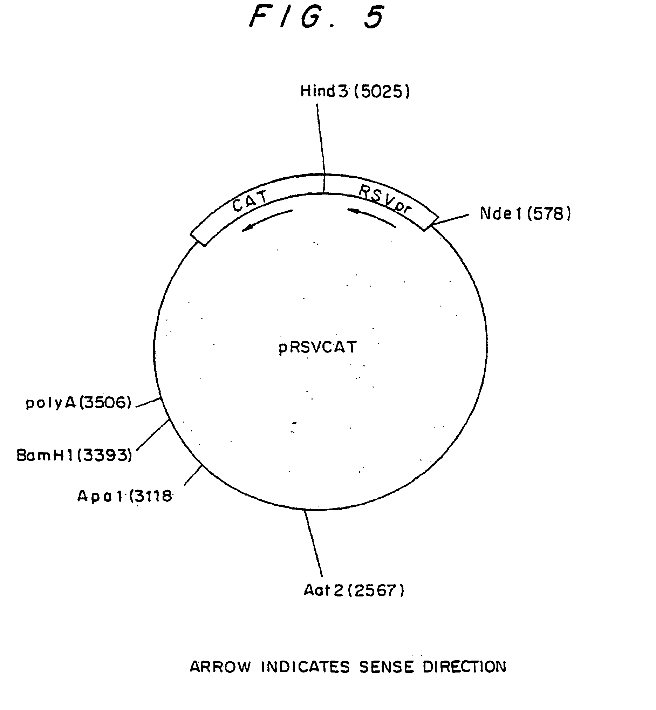

Fig 5 shows the pRSVCAT plasmid, including the restriction sites thereof.

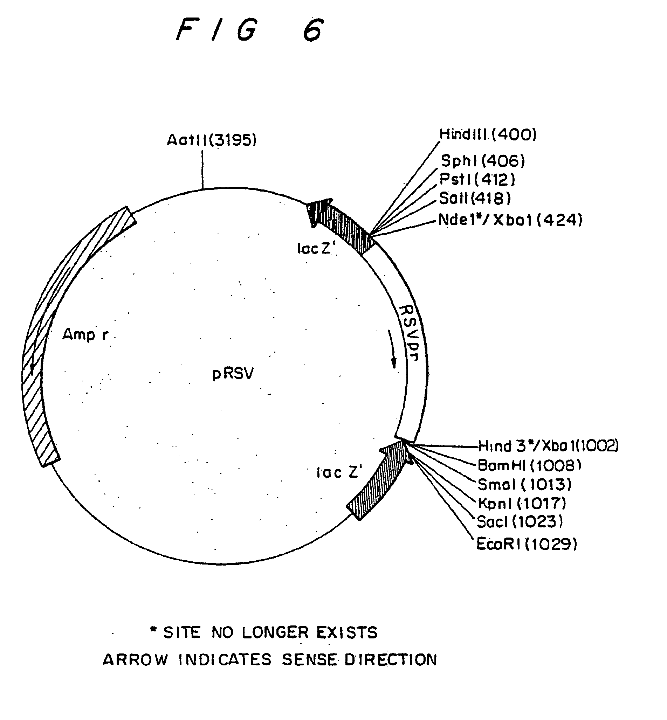

Fig. 6 shows the construction of the pRSV plasmid, including the restriction sites thereof.

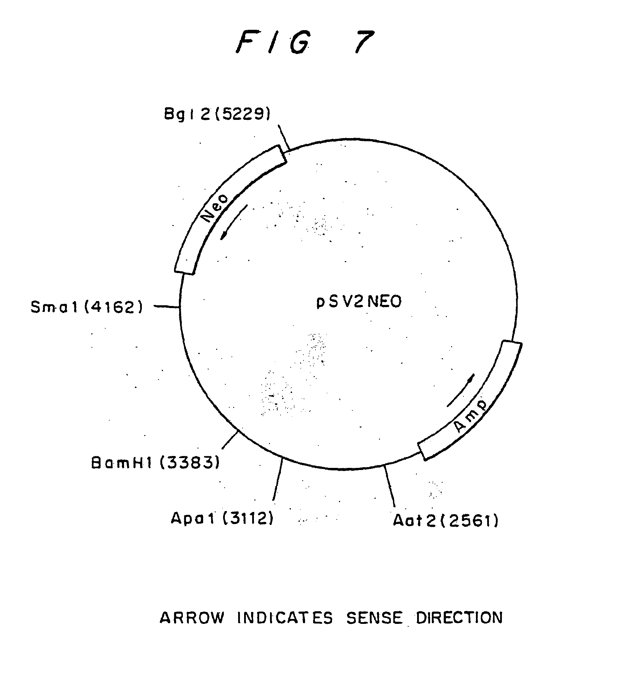

Fig. 7 shows the pSV2NEO plasmid, including the restriction sites thereof.

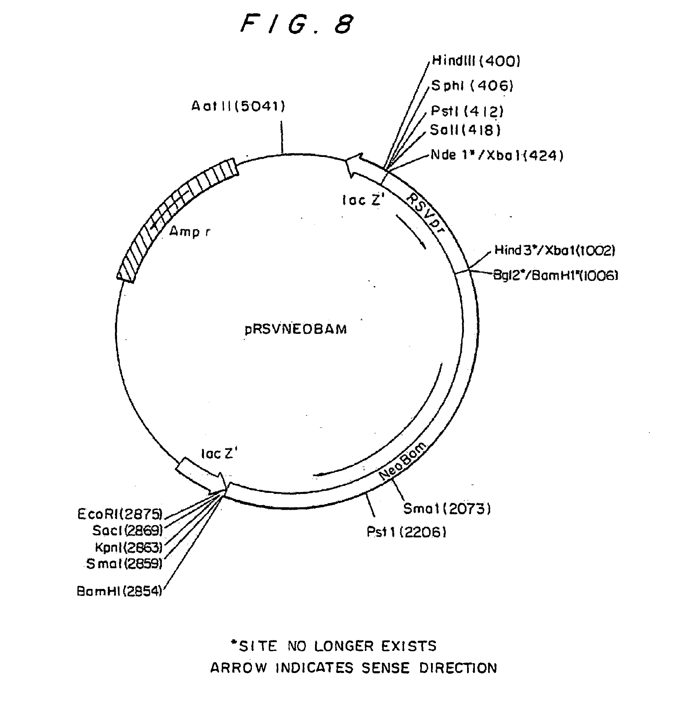

Fig. 8 shows the construction of the pSVNEOBAM plasmid, including the restriction sites thereof.

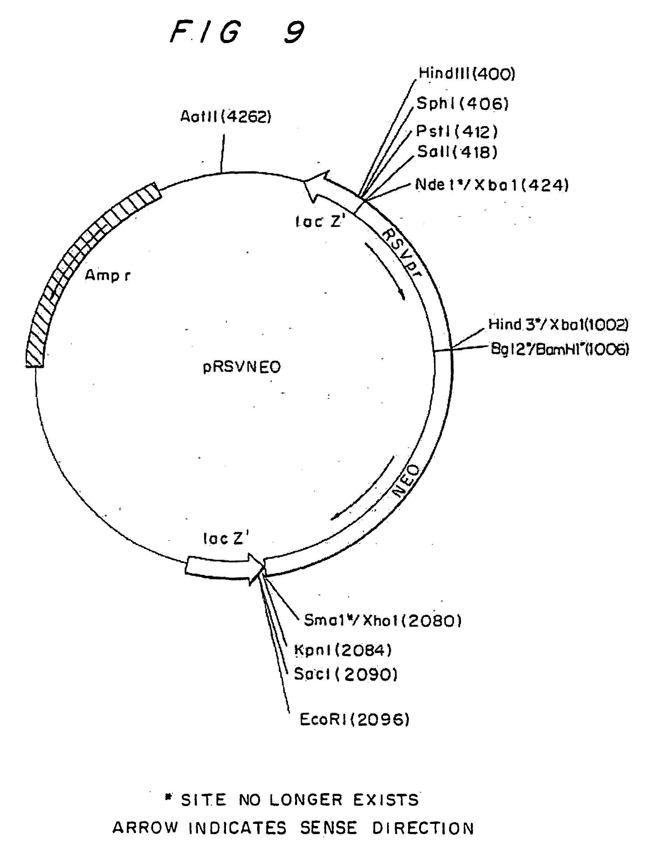

Fig. 9 shows the construction of the pRSVNEO plasmid, including the restriction sites thereof.

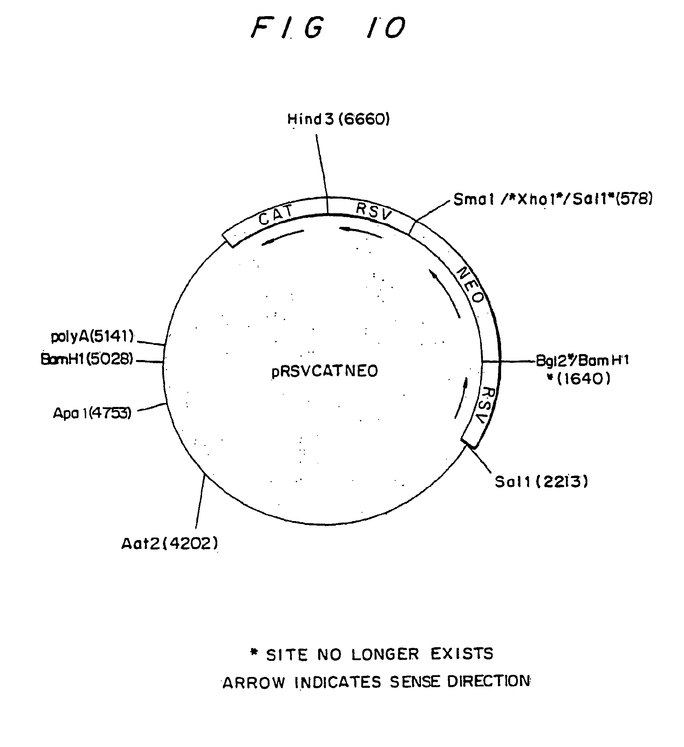

Fig. 10 shows the construction of the pRSVCATNEO plasmid, including the restriction sites thereof.

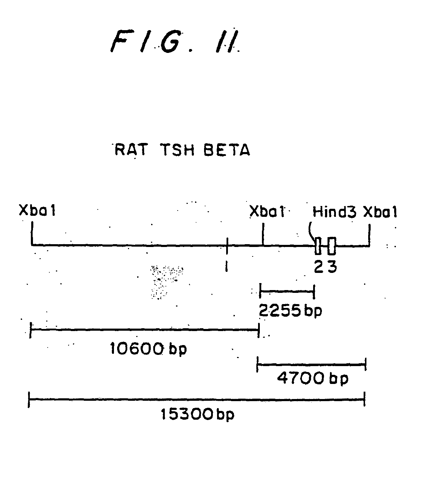

Fig. 11 shows a 15.3 kb fragment of the rat TSHb gene and showing various restriction segments thereof.

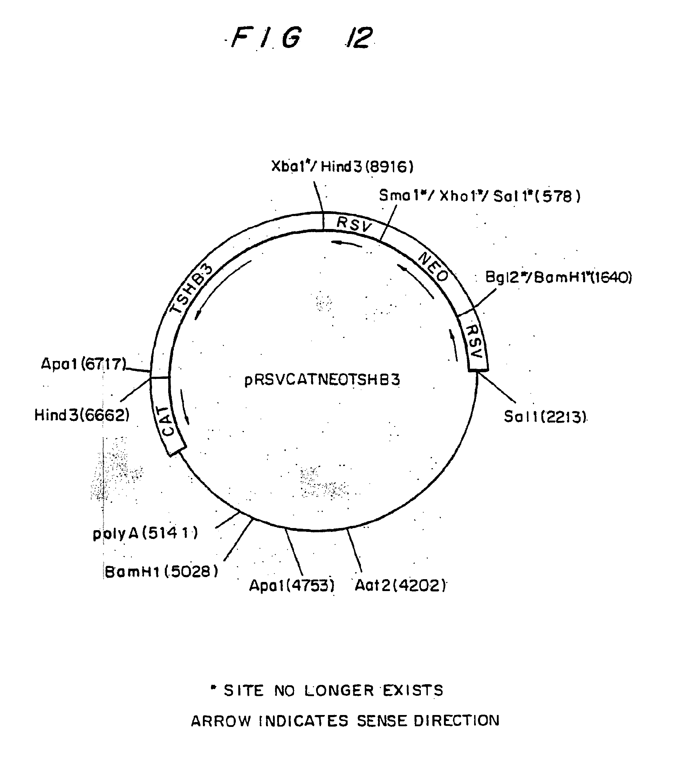

Fig. 12 shows the construction of the pRSVCATNEOTSHB3 plasmid, including the restriction sites thereof.

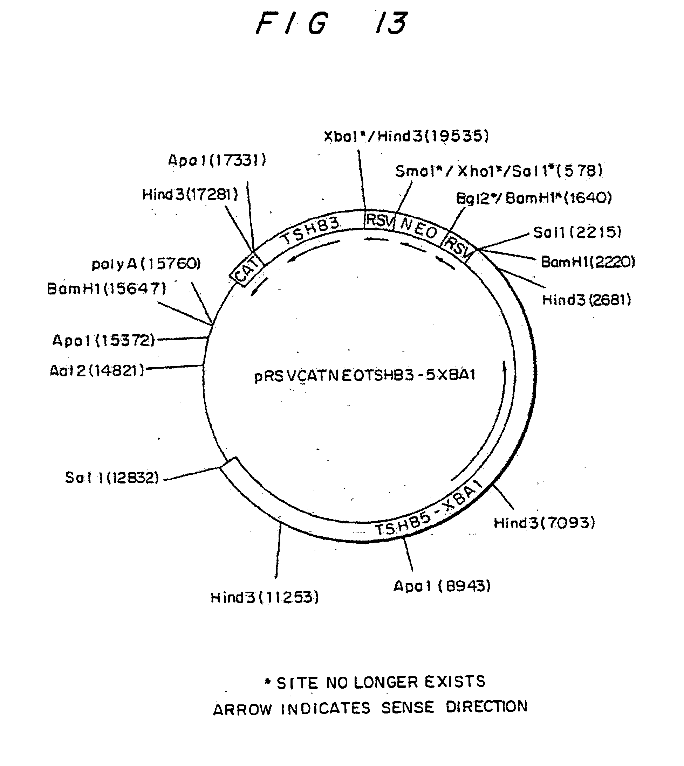

Fig. 13 shows the construction of the pRSVCATNEOTSHB3-5XbaI plasmid, including the restriction sites thereof.

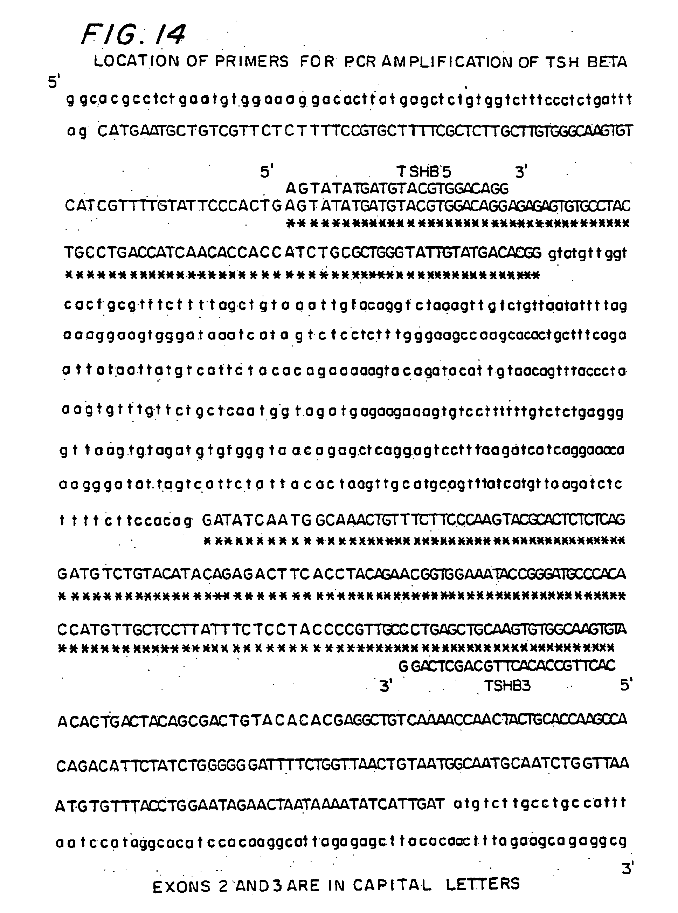

Fig. 14 shows a portion of the nucleotide sequence of TSHb along with the regions thereof to which each primer for PCR amplification corresponds. Exons 2 and 3 are shown in capital letters. A 247 BP amplified fragment is shown by underlined asterisks.

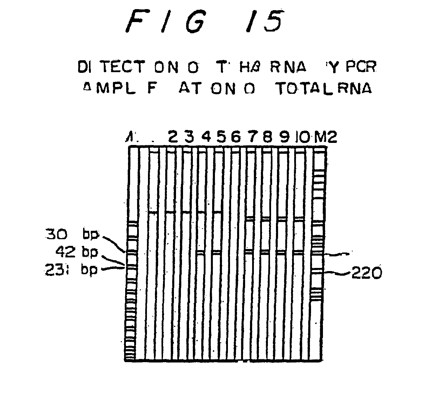

Fig. 15 shows the results of polyacrylamide gel electrophoresis of cDNA synthesized from RNA extracted from various cell populations and whose TSHb cDNA, if present, has been amplified by PCR. The nature of the cells representing the various lanes is set forth in Fig. 15 below the gel.

### DETAILED DESCRIPTION OF PREFERRED EMBODIMENTS

Homologous recombination is a technique developed within the past few years for targeting genes to induce or correct mutations in transcriptionally active genes (Kucherlapati, Prop. in Nucl. Acid Res. and Mol. Biol., 36:301 (1989)). This technique of homologous recombination was developed as a method for introduction of specific mutations into specific regions of the mammalian genome (Thomas et al., Cell, 44:419-428, 1986; Thomas and Capecchi, Cell, 51:503-512, 1987; Doetschman et al., Proc. Natl. Acad. Sci., 85:8583-8587, 1988) or to correct specific mutations within defective genes (Doetschman et al., Nature, 330:576-578, 1987).

Through this technique, a piece of DNA that one desires to be inserted into the genome can be directed to a specific region of the gene of interest by attaching it to "targeting DNA". "Targeting DNA" is DNA that is complementary (homologous) to a region of the genomic DNA. If two homologous pieces of single stranded DNA (i.e., the targeting DNA and the genomic DNA) are in close proximity, they will hybridize to form a double stranded helix. Attached to the targeting DNA is the DNA sequence that one desires to insert into the genome.

There are a number of methods by which homologous recombination can occur. One example is during the process of replication of DNA during mitosis in cells.

Through a mechanism that is not completely understood, parental double-stranded DNA is opened immediately prior to cell division at a local region called the replication bubble. The two separated strands of DNA may now serve as templates from which new strands of DNA are synthesized. One arm of the replication fork has the DNA code in the 5' to 3' direction, which is the appropriate orientation from which the enzyme DNA polymerase can "read". This enzyme attaches to the 5' portion of the single stranded DNA and using the strand as a template, begins to synthesize the complementary DNA strand. The other parental strand of DNA is encoded in the 3' to 5' direction. It cannot be read in this direction by DNA polymerase. For this strand of DNA to replicate, a special mechanism must occur.

A specialized enzyme, RNA primase, attaches itself to the 3' to 5' strand of DNA and synthesizes a short RNA primer at intervals along the strand. Using these RNA segments as primers, the DNA polymerase now attaches to the primed DNA and synthesizes a complementary piece of DNA in the 5' to 3' direction. These pieces of newly synthesized DNA are called Okazaki fragments. The RNA primers that were responsible for starting the entire reaction are removed by the exonuclease function of the DNA polymerase and replaced with DNA. This phenomenon continues until the polymerase reaches an unprimed stretch of DNA, where the local synthetic process stops. Thus, although the complementary parental strand is synthesized overall in the 3' to 5' direction, it is actually produced by "backstitching" in the 5' to 3' direction. Any nicks that might occur in the DNA during the "backstitching" process are sealed by an enzyme called DNA ligase.

To maintain an absolute fidelity of the DNA code, a proofreading function is present within the DNA polymerase. The DNA polymerase requires primed pieces of DNA upon which to synthesize a new strand of DNA. As mentioned above, this can be a single strand of DNA primed with RNA, or a complementary strand of DNA. When the DNA polymerase finds mismatched complementary pieces of DNA, it can act as an exonuclease and remove DNA bases in a 3' to 5' direction until it reaches perfect matching again.

With this background, it is now possible to understand the basis of the technique described herein. Small pieces of targeting DNA that are complementary to a specific region of the genome are put in contact with the parental strand during the DNA replication process. It is a general property of DNA that has been inserted into a cell to hybridize and therefore recombine with other pieces of DNA through shared homologous regions. If this complementary strand is attached to an oligonucleotide that contains a mutation or a different sequence of DNA, it too is incorporated into the newly synthesized strand as a result of the recombination. As a result of the proofreading function, it is possible for the new sequence of DNA to serve as the template. Thus, the transfected DNA is incorporated into the genome.

If the sequence of a particular gene is known, a piece of DNA that is complementary to a selected region of the gene can be synthesized or otherwise obtained, such as by appropriate restriction of the native DNA at specific recognition sites bounding the region of interest. This piece will act as a targeting device upon insertion into the cell and will hybridize to its homologous region within the genome. If this hybridization occurs during DNA replication, this piece of DNA, and any additional sequence attached thereto, will act as an Okazaki fragment and will be backstitched into the newly synthesized daughter strand of DNA.

In the technique of the present invention, attached to these pieces of targeting DNA are regions of DNA that are known to interact with the nuclear regulatory proteins present within the cell and, optionally, amplifiable and selectable DNA markers. Thus, the expression of specific proteins may be achieved not by transfection of DNA that encodes the gene itself and marker DNA, as is most common, but rather by the use of targeting DNA (regions of homology with the endogenous gene of interest) coupled with DNA regulatory segments that provide the gene with recognizable signals for transcription. With this technology, it is possible to express and to amplify any cognate gene present within a cell type without actually transfecting that gene. In addition, the expression of this gene is controlled by the entire genomic DNA rather than portions of the gene or the cDNA, thus improving the rate of transcription and efficiency of mRNA processing. Furthermore, the expression characteristics of any cognate gene present within a cell type can be modified by appropriate insertion of DNA regulatory segments and without inserting entire coding portions of the gene of interest.

In accordance with these aspects of the instant invention there are provided new methods for expressing normally transcriptionally silent genes of interest, or for modifying the expression of endogenously expressing genes of interest1 within a differentiated cell line. The cognate genomic sequences that are desired to be expressed, or to have their expression modified, will be provided with the necessary cell specific DNA sequences (regulatory and/or amplification segments) to direct or modify expression of the gene within the cell. The resulting DNA will comprise the DNA sequence coding for the desired protein directly linked in an operative way to heterologous (for the cognate DNA sequence) regulatory and/or amplification segments; A positive selectable marker is optionally included within the construction to facilitate the screening of resultant cells. The use of the neomycin resistance gene is preferred, although any selectable marker may be employed. Negative selectable markers may, optionally, also be employed. For instance, the Herpes Simplex Virus thymidine kinase (HSVtk) gene may be used as a marker to select against randomly integrated vector DNA. The fused DNAs, or existing expressing DNAs, can be amplified if the targeting DNA is linked to an amplifiable marker.

Therefore, in accordance with the method of the present invention, any gene which is normally expressed when present in its specific eukaryotic cell line, particularly a differentiated cell line, can be forced to expression in a cell line not specific for it wherein the gene is in a silent format. This occurs without actually inserting the full DNA sequence for that gene. In addition, that gene, or a normally expressing gene, can be amplified for enhanced expression rates. Furthermore, the expression characteristics of genes not totally transcriptionally silent can be modified as can the expression characteristics of genes in microorganisms.

In one embodiment of the present invention, eukaryotic cells that contain but do not normally transcribe a specific gene of interest are induced to do so by the technique described herein. The homologous recombination vector described below is inserted into a clonal cell line and, following chemical selection, is monitored for production of a specific gene product by any appropriate means, such as, for example, by detection of mRNA transcribed from the newly activated gene, immunological detection of the specific gene product, or functional assay for the specific gene product.

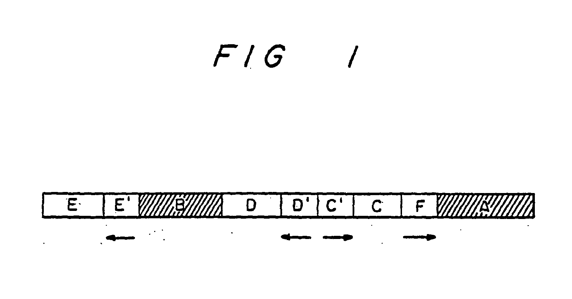

The general outline of the DNA construct that is used to transcriptionally activate endogenous genes by homologous recombination is depicted in Figure 1.

In general, the DNA construct comprises at least two and up to six or more separate DNA segments. The segments consist of at least one, preferably two, DNA targeting segments (A and B) homologous to a region of the cell genome within or proximal to the gene desired to be expressed, a positive selection gene (C), an amplifiable gene (D), a negative selection gene (E) and a DNA regulatory segment (F) which is transcriptionally active in the cell to be transfected. In the most basic embodiment of the present invention, only a single targeting segment (B) and the regulatory segment (F) must be present. All of the other regions are optional and produce preferred constructs.

Regions A and B are DNA sequences which are homologous to regions of the endogenous gene of interest which is to be made transcriptionally active. The specific regions A and B of the endogenous gene are selected so as to be upstream and downstream, respectively, of the specific position at which it is desired for the regulatory segment to be inserted. Although these regions are separated in the construct they are preferably contiguous in the endogenous gene. There may be occasions where noncontiguous portions of the genome are utilized as targeting segments, for example, where it is desired to delete a portion of the genome, such as a negative regulatory element.

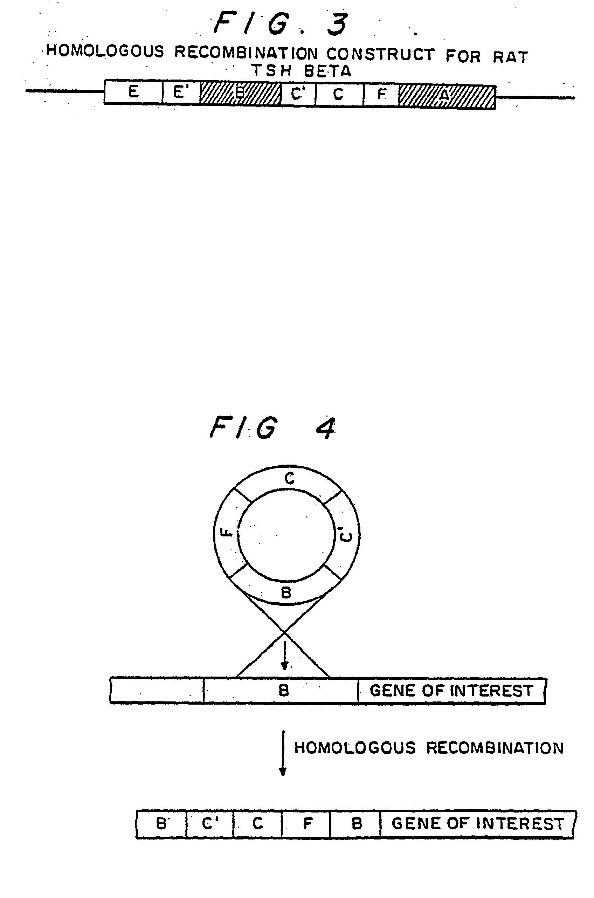

While two targeting regions, A and B, are preferred in order to increase the total regions of homology and thus increase recombination efficiency, the process of the present invention also comprehends the use of only a single targeting region. In its simplest form (when only the regulatory segment F and the selectable marker gene C and promoter C' are to be inserted), a circular piece of DNA is employed which contains these elements along with the targeting DNA (see Figure 4). In this way, the homologous region (B) hybridizes with its genomic counterpart. Segments C', C and F are inserted within the B portion of the cognate gene following the crossover event.

When it is desired for the DNA regulatory sequence to be inserted upstream of the gene of interest, as, for example, when it is desired to activate and express a normally transcriptionally silent gene, the region of homology is preferably homologous to a non-coding portion of the genome upstream of the coding portions of the gene of interest. When two targeting regions are present, the downstream region (A) may include a portion of the coding region, although it is preferred that it, too, be totally upstream of the coding region. It is further preferred that the homologous regions be chosen such that the DNA regulatory sequence will be inserted downstream of the native promoter for the gene of interest, particularly if the native promoter is a negative promoter rather than a turned-off positive promoter.

The size of the targeting regions, i.e., the regions of homology, is not critical, although the shorter the regions the less likely that they will find the appropriate regions of homology and recombine at the desired spot. Thus, the shorter the regions of homology, the less efficient is the homologous recombination, i.e., the smaller the percentage of successfully recombined clones. It has been suggested that the minimum requirement for sequence homology is 25 base pairs (Ayares et al, PNAS. USA, 83:5199-5203, 1986). Furthermore, if any of the other elements of the construct are also found in the genome of the host cell, there is a possibility of recombination at the wrong place. However, in view of the excellent positive and negative selectability of the present invention, it can be successfully practiced even if the efficiency is low. The optimum results are achieved when the total region of homology, including both targeting regions, is large, for example one to three kilobases. As long as the regulatable segment F can be operatively linked to the gene of interest there is no limit to the size of the targeting region, and particularly the upstream targeting region B.

It can easily be empirically determined whether or not the targeting regions are too large or the regulatable segment F spaced too far from the coding region of the gene to be operatively linked thereto. In such a case, the regions A and B can be made homologous to a different section of the gene of interest and the process repeated until the regulatable segment F is properly inserted so as to be operatively linked to the gene of interest. For example, the restriction site of combined region A-B of the endogenous gene can be changed and the process repeated. Once the concept of the present invention is known, along with the techniques disclosed herein, one of ordinary skill in this art would be able to make and use the present invention with respect to any given gene of interest in any cell line or microorganism without use of undue experimentation.

Region C is a positive selectable marker gene which is capable of rendering the transfected cell line resistant to a normally toxic environment. Examples of such genes are adenosine deaminase (ADA), aminoglycoside phosphotransferase (neo), dihydrofolate reductase (DHFR), hygromycin-B-phosphotransferase (HPH), thymidine kinase (tk), xanthine-guanine phosphoribosyltransferase (gpt), multiple drug resistance gene (MDR), ornithine decarboxylase (ODC) and N-(phosphonacetyl) -L-aspartate resistance (CAD).

In addition to the positive selectable marker gene, an amplifiable gene is also optionally included in the construct at region D. Amplifiable genes are genes that lead to an increase in copy number when under selective pressure. The copy number of a gene positioned adjacent to the amplifiable gene will also increase. Amplifiable genes that can be utilized include DHFR, MDR, ODC, ADA and CAD. The members of the positive selectable marker gene group and those of the amplifiable gene group overlap so that, in theory, instead of using two genes, one for positive selection and one for amplification, one gene could be used for both purposes. However, since most cell lines contain endogenous copies of these amplifiable genes, the cells will already be somewhat resistant to the selection conditions and distinguishing the cells which have transfected DNA from those which do not receive transfected DNA can be difficult. Thus, in instances where an amplifiable gene is desired, a positive selection gene which is dominant, such as HPH, gpt, neo and tk (in tk cells), should also be included in the construct. For some applications it may be possible or preferable to omit the amplifiable marker. For instance, the gene of interest may not need to be amplified as, for example, when transcriptional activation by the heterologous DNA regulatory sequence is sufficient without amplification. Also, if the homologous recombination efficiency is very low, it may be necessary to leave out the amplifiable gene since the ratio of non-homologous DNA to homologous DNA is directly related to the homologous recombination efficiency (Letsou, Genetics, i17:759-770, 1987). It is also possible to eliminate the positive selection gene and select cells solely by screening for the production of the desired protein or mRNA. However, it is preferred in most cases to include at least the positive selection gene.

Region E of the construct is a negative selectable marker gene. Such a gene is not expressed in cells in which the DNA construct is properly inserted by homologous recombination, but is expressed in cells in which the DNA construct is inserted improperly, such as by random integration. One such gene is the Herpes Simplex Virus thymidine kinase gene (HSVtk). The HSVtk has a lower stringency for nucleotides and is able to phosphorylate nucleotide analogs that normal mammalian cells are unable to phosphorylate. If the HSVtk is present in the cells, nucleotide analogs such as acyclovir and gancyclovir are phosphorylated and incorporated into the DNA of the host cell thus killing the cell. The presence of the negative selectable marker gene enables one to use the positive-negative selection for homologous recombination as described by Mansour et al (Nature, 336:348-352, 1988). Capecchi uses a strategy which takes advantage of the differing modes of integration that occur when linearized vector DNA inserts via homologous recombination as compared to when it inserts by random integration. If the vector DNA inserts randomly, the majority of the inserts will insert via the ends (Folger et al, Mol. Cell Biol., 2:1372-1387, 1982; Roth et al, Mol. Cell. Biol., 5:25992607, 1985; and Thomas et al, Cell, 44:419-428, 1986). On the other hand, if the vector inserts by homologous recombination, it will recombine through the regions of homology which cause the loss of sequences outside of those regions.

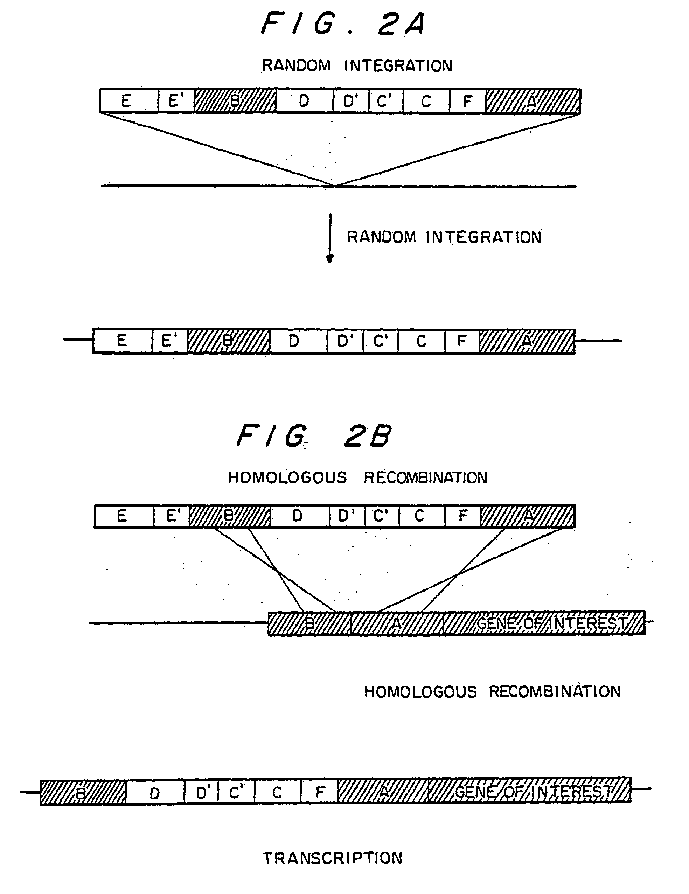

Using the construct depicted in Figure 1 as an example, the mode of integration for homologous recombination versus random integration is illustrated in Figures 2A and 2B. In the case of non-homologous recombination (Figure 2A), the vector is inserted via the ends of the construct. This allows region E, in this case the HSVtk gene, to be inserted into the genome. However, when homologous recombination occurs (Figure 2B), the HSVtk gene is lost. The first round of selection uses the appropriate drug or conditions for the positive selection present within the construct. Cells which have DNA integrated either by homologous recombination or random integration will survive this round of selection. The surviving cells are then exposed to a drug such as gancyclovir which will kill all the cells that contain the HSVtk gene. In this case, most of the cells in which the vector integrated via a random insertion contain the HSVtk gene and are killed by the drug while those in which the vector integrated by homologous recombination have lost the HSVtk gene and survive. This allows the elimination of most of the cells which contain randomly integrated DNA, leaving the majority of the surviving cells containing DNA which integrated via homologous recombination. This greatly facilitates identification of the correct recombination event.

The negative selection step can also be eliminated if necessary. It will require that the screening step be more labor intensive involving the need for techniques such as polymerase chain reaction (PCR) or immunological screening.

The sixth region (F) contains the DNA regulatory segment that will be used to make the gene of interest transcriptionally active. The appropriate DNA regulatory segment is selected depending upon the cell type to be used. The regulatory segment preferably used is one which is known to promote expression of a given gene in differentiated host cell line. For example, if the host cell line consists of pituitary cells which naturally express proteins such as growth hormone and prolactin, the promoter for either of these genes can be used as DNA regulatory element F. When inserted in accordance with the present invention, the regulatory segment will be operatively linked to the normally transcriptionally silent gene of interest and will stimulate the transcription and/or expression of that gene in the host cell line. Also usable are promiscuous DNA regulatory segments that work across cell types, such as the rous sarcoma virus (RSV) promoter. As long as the regulatory segment stimulates transcription and/or expression, or can be induced to stimulate transcription and/or expression, of the gene of interest after being inserted into the host cell line so as to be operatively linked to the gene of interest by means of the present invention, it can be used in the present invention. It is important when joining the regulatory segment F to the targeting segment A that no starting codon be accidentally introduced into the sequence since such an occurrence could alter the reading frame of the gene which is desired to be expressed. Of course, the construct must be constructed and inserted such that the regulatory segment F is operatively linked to the gene of interest.

The DNA regulatory segment, region F, need not be present in instances where it is desired to enhance or amplify the transcription of a gene which is already expressing in the cell line of interest, either because it naturally expresses in that cell line or because the cell line has previously had its DNA manipulated to cause such expression. In such instances, insertion of an amplifiable gene, region D, preferably with the positive selectable marker gene, region C, and optionally also with a negative selectable marker gene, region E, will be sufficient to increase the copy number of the gene of interest and thus enhance the overall amount of transcription. Alternatively, a new regulatory segment, region F, inherently promoting an increased (or otherwise modified) rate of transcription as compared to the existing regulatory region for the gene of interest, may be included to further enhance the transcription of the existing expressing gene of interest. Such a new regulatory segment could include promoters or enhancers which improve transcription efficiency.

Regions C', D' and E' are promoter regions which are used to drive the genes in regions C, D, and E, respectively. These promoters are transcriptionally active in the cell line chosen and may be the same or different from the promoter in region F used to drive the endogenous gene of interest. The specific direction of transcription specified in Fig. 1 is not critical. Those of ordinary skill in this art can determine any appropriate placement of the genes C, D and E and their promoters C', D' and E' such that the promoters will stimulate expression of their associated genes without simultaneously disrupting in any way the expression of the gene of interest or any of the other genes of the construct.

The present invention is not limited to the cell line that is described herein. All cell lines have genetic information which is normally silent or inert. Most are able to express only certain genes. However, a normally transcriptionally silent or inert gene of any such cell line can be activated to express the gene product in accordance with the present invention and any gene of the genome may have its expression characteristics modified in accordance with the present invention. Even previously transformed cell lines can be used as long as the previous transformation did not disrupt the gene of interest. The source of the cell line is not important. The cell line may be animal or plant, primary, continuous or immortal. Of course, it is desirable that any such cell line be stable and immortal so that after treatment with the technique in accordance with the present invention, expression can be commercialized. Cloned microorganisms, whether prokaryotic or eukaryotic, may also be treated by the technique of the present invention.

While the present invention has been preferably described with respect to the expression of a normally transcriptionally silent or inert gene, the technique of the present invention is also applicable to the modification of the expression characteristics of a gene which is naturally expressed in the host cell line. For example, if it is desired to render the expression of a gene dependent upon culture conditions or the like so that expression can be turned on and off at will, an appropriate DNA regulatory segment, such as a regulatable promoter, can be inserted which imparts such characteristics, such as repressibility or inducibility. For example, if it is known that the cell type contains nuclear steroid receptors, such as estrogen, testosterone or glucocorticoid, or thyroxin receptors, one could use the steroid or thyroxin response elements as region F. Such a response element is any DNA which binds such receptor to elicit a positive response relative to transcription. Even if a cell is not naturally responsive to glucocorticoids, for example, a piece of DNA which encodes the glucocorticoid receptor could be added to the construct, or otherwise inserted somewhere in the genome, so as to make the cell responsive to glucocorticoids. The use of a regulatable promoter could be desirable whether or not the gene of interest is normally transcriptionally silent. Other kinds of regulation can also be obtained by targeting the appropriate DNA regulatory segment to the exact position of interest by means of the process of the present invention.

Thus, while stimulation of expression of normally transcriptionally silent genes is the preferred application of the present invention, in its broadest sense it is applicable to the modification of expression characteristics of any gene endogenous to the host cell line.

The specific technique of homologous recombination is not, per se, a novel part of the present invention. Such techniques are known and those of ordinary skill in this art will understand that any such technique can be used in the present invention as long as it permits targeting of the DNA regulatory sequence to the desired location with respect to the gene of interest. While a preferred technique is disclosed, using a linearized construct with two homologous regions on either end of the sequences to be inserted, any other technique which will accomplish this function, as, for example, by using circular constructs, is also intended to be comprehended by the present invention. The critical feature of the present invention is the use of homologous recombination techniques to insert a DNA regulatory sequence which causes modification of expression characteristics in the cell line or microorganism being used, operatively linked with a gene in the genome of the cell line, preferably one which is normally transcriptionally silent, or to insert an amplifiable sequence, without a regulatory sequence, sufficiently near a gene in the genome of the cell line which already transcribes as to cause amplification of such gene upon amplification of the amplifiable sequence. It is not absolutely necessary that a selectable marker also be included. Selection can be based solely on detection of the gene product of interest or mRNAs in the media or cells following insertion of the DNA construct. Furthermore, in the embodiment in which a regulatory sequence is being inserted, amplification, while desired, is not critical for operability. The same is true for the negative selection gene which makes the screening process easier, but is not critical for the success of the invention. Thus, the basic embodiment requires only insertion of the DNA regulatory segment or the amplifiable segment in the specific position desired. However, the addition of positive and/or negative selectable marker genes for use in the selection technique is preferred, as is the addition of an amplifiable gene in the embodiment in which a regulatory segment is being added.

The term "modification of expression" as used throughout the present specification and claims, is hereby defined as excluding termination of expression by inserting by homologous recombination a mutation, deletion, stop codon, or other nucleotide sequence, including an entire gene, into the gene of interest, so as to prevent the product of interest from being expressed. The prior art teaches the use of homologous recombination to insert specific mutations and the expression of a cell product may have inherently been terminated by means thereof (see, for example, Schwartzberg et al, PNAS (USA), 87:3210-3214 (1990)). The present invention is not intended to encompass such a procedure. In the present invention the "modification of expression" is accomplished by means of inserting regulatory and/or amplification regions at a specific desired location by means of homologous recombination. The preferred modifications are those which activate and/or enhance expression of the product of interest.

Whenever the present specification uses the phrase that a DNA regulatory segment is "operatively linked with" a gene, such terminology is intended to mean that the DNA regulatory segment is so disposed with respect to the gene of interest that transcription of such gene is regulated by that DNA regulatory segment. The regulatory segment is preferably upstream of the gene, but may be downstream or within the gene, provided that it operates to regulate expression of the gene in some way. The DNA regulatory segment may be a promoter, terminator, operator, enhancer, silencer, attenuator, or the like, or any combination thereof.

Whenever the terms "upstream" or "downstream" are used in the present specification and claims, this is intended to mean in the 5'-direction or the 3'-direction, respectively, relative to the coding strand of the gene of interest.

The foregoing description of the specific embodiments so fully reveals the general nature of the invention that others can readily modify and/or adapt such specific embodiments for various applications without departing from the generic concept. Any such adaptations and modifications are intended to be embraced within the meaning and range of equivalents of the disclosed embodiments. It is to be understood that the phraseology and terminology employed herein are for the purpose of description and not of limitation.

## Claims

1. A method for producing a clonal eukaryotic cell line comprising:
- inserting by homologues recombination a DNA construct comprising a selectable marker, at least a DNA regulatory segment (F) which activates or modifies expression of a gene in a eukaryotic host cell when operatively linked thereto, two DNA targeting segments (A, B) homologous to a region of the genome within or proximal to the gene within the host cell line, one of which (A) is homologous to a region of the genome located downstream of the specific region at which the regulatory segment (F) is inserted, and the other of which (B) is homologous to a region of the genome located upstream of the specific region at which the regulatory segment (F) is inserted,; said method further comprising
- chemical selection; and
- monitoring for production of the specific gene product in the clonal eukaryotic host cell line.

2. A clonal eukaryotic cell line obtainable according to claim 1.

3. Method for the preparation of a desired product by culturing the cell of claim 2.
